Europäisches Patentamt

European Patent Office

Office européen des brevets

(11) Publication number: **0 205 306 B1**

# EUROPEAN PATENT SPECIFICATION

(45) Date of publication of patent specification: 18.12.91   (51) Int. Cl.⁵: **A61K 7/11**

(21) Application number: 86304230.5

(22) Date of filing: 04.06.86

The file contains technical information submitted after the application was filed and not included in this specification

(54) **Hair styling mousse and method.**

(30) Priority: 06.06.85 US 741770
06.03.86 US 836794

(43) Date of publication of application:
17.12.86 Bulletin 86/51

(45) Publication of the grant of the patent:
18.12.91 Bulletin 91/51

(84) Designated Contracting States:
AT BE CH DE FR IT LI LU NL SE

(56) References cited:
GB-A- 2 132 653
US-A- 3 655 865

SOAP, PERFUMERY & COSMETICS, vol. 36, no. 11, November 1963, pages 997-1002; "Focus on Aerosols"

RIECHSTOFFE, AROMEN, KÖRPERP-FLEGEMITTEL, vol. 21, no. 10, October 1971, page 374; "Haarspray mit Antischuppenwir-kung", "Haarspray normal", "Haarspray, hart", "Haarfestiger, normal", "Haarfestiger, hart"

CHEMICAL ABSTRACTS, vol. 86, no. 26, 27th

June 1977, page 318, abstract no. 195065f, Columbus, Ohio, US; & JP-A-77 05 683 (KANEBO LTD) 17-01-1977

(73) Proprietor: THE PROCTER & GAMBLE COM-PANY
One Procter & Gamble Plaza
Cincinnati Ohio 45202(US)

(72) Inventor: **Bolich, Raymond Edward, Jr.**
7201 Striker Road
Mainville OH 45039(US)

(74) Representative: **Brooks, Maxim Courtney et al**
Procter & Gamble (NTC) Limited Whitley
Road Longbenton
Newcastle-upon-Tyne NE12 9TS(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to aqueous hair stying aerosol mousse compositions which provide excellent set hold as well as combing ease.

The desire to have hair (human or other animal) retain a particular shape or configuration is one that is widely held. Approaches taken can either involve permanent alteration of the hair or a temporary alteration. The former involves the use of chemical agents to react with the hair in order to achieve the desired effect. This process can be carried out at either room or elevated temperature.

The temporatory set given to hair is, as the term indicates, a temporary arrangement which can be removed by water or by shampooing. The materials used to provide the set have generally been resins or gums. The temporary set compositions have taken the form of gels, lotions and sprays as well as others. The compositions are applied most often to hair dampened with water, combed or by other means spread through the hair and let dry. The set given will vary depending on the materials used.

In recent years a form of a temporary set has been achieved by means of an aerosol foam -- a mousse. This form, which can easily be worked through the hair, can provide a set comparable to that given by a gel or a lotion. These products are generally applied to the user's hand and worked through the hair.

The conventional hair styling mousse, which got its start in Europe, generally utilizes a water soluble polymer, water, possibly a conditioning agent, an emulsifier, aesthetic agents and the propellant. The conditioning agents used have included silicone type materials. Such formulations are disclosed in Billek, Doris E., "Aerosol Foam and Mousse Preparations in Europe", Cosmetics & Toiletries, Vol. 99 (September 1984), 57-60, 62-67.

The present invention involves the use of high molecular weight silicone materials in styling mousses. The formulae disclosed in Billek, while containing silicone type agents, do not disclose high molecular weight materials or any materials dissolved in the propellant phase. A reference disclosing a variety of materials dissolved in the propellant phase prior to filling the container is DE-A-2,943,521. This reference does not however disclose high molecular weight silicone materials either. Such materials are however disclosed in non-aerosol hair conditioning formulations in US-A-4,387,090. Another reference disclosing the use of low molecular weight silicone materials in aerosol compositions is US-A-3,655,865.

The present inventor has found that combining the silicone material with the propellant eliminates the need to mill the material, which would probably be necessary if it were to be combined with the aqueous phase. Additionally unexpected results have been found in hair conditioning (dry combing) and set hold when the material is in the propellant phase.

Therefore it is a purpose of the present invention to provide a superior hair styling mousse.

It is a further object of the present invention to provide a hair styling mousse employing a high molecular weight silicone material dissolved in the propellant phase.

These and other objects of the present invention will become readily apparent from the detailed description which follows.

All percentages and ratios used herein are by weight unless otherwise specified.

Accordingly, the present invention provides an aerosol hair styling mousse comprising:

a) from 0.5% to 10% of a hair setting polymer;

b) from 0.05% to 2% of a high molecular weight silicone material;

c) from 5% to 20% of an aerosol propellant; and

d) water;

wherein said high molecular weight silicone material has a viscosity of at least 100,000 $mm^2.s^{-1}$ (cSt) and wherein said silicone material is dissolved in said propellant prior to said propellant being put into an aerosol container.

The essential as well as optional components are specified below.

### Water

Water, preferably distilled or deionized, is the first essential component of the present invention. The water is generally present at a level of from 55% to 94%, preferably from 80% to 90% of the total composition.

### Polymer

The polymer useful in the present compositions is any polymer soluble or colloidally dispersible in the aqueous phase (if water is the only solvent in the aqueous phase, the polymer should be soluble or dispersible in water; if an optional cosolvent such as ethanol is present the polymer should be soluble or dispersible in the combined solvent system). Solubility/ dispersibility is determined at ambient conditions (e.g., temperature about 25°C and atmospheric pressure). Suitable types of polymers include anionic, nonionic, amphoteric and cationic. Specific polymers include polyvinylpyrrolidone (PVP), copolymers of (PVP) and methylmethacrylate, copolymers of PVP and vinylacetate (VA), polyvinyl alcohol (PVA), copolymers of PVA and crotonic acid, copolymers of PVA and maleic anhydride, hydroxypropyl cellulose, hydroxypropyl guar gum, sodium polystyrene sulfonate, PVP/ ethylmethacrylate/methacrylic acid terpolymer and octylacrylamide/acrylate/butylaminoethyl methacrylate copolymers. PVP and PVP copolymers with other monomers are preferred.

Mixtures of polymers may also be used. With certain of the polymers it may be necessary to neutralize some acidic groups to promote solubility/dispersibility (e.g., PVA/crotonic acid).

The polymer(s) is used at a level of from 0.5% to 10%, preferably from 1% to 6% of the total composition. The mass average molecular weight of the polymer is not critical but is generally in the range of from about 2,000 to about 2,000,000.

### Propellant

The agent responsible for expelling the other materials from the container and forming the mousse character is a propellant.

The propellant gas can be any liquefiable gas conventionally used for aerosol containers. Preferably the density of the propellant or mixture thereof is less than 1 so that pure propellant is not emitted from the container. Examples of materials that are suitable for use as propellants are trichlorofluoromethane, dichlorodifluoromethane, dichlorotetrafluoroethane, monochlorodifluoromethane, trichlorotrifluoroethane, dimethylether, propane, $\eta$-butane and isobutane, used singly or admixed. The hydrocarbons, particularly isobutane, used singly or admixed with other hydrocarbons, are preferred due to their densities being less than 1.

The amount of the propellant gas is governed by normal factors well known in the aerosol art. For mousses the level of propellant is from 5% to 20%, preferably from 7% to 15% of the total composition. If a propellant such as dimethylether utilizes a vapor pressure suppressant (e.g., trichloroethane or dichloromethane) the amount of suppressant is included as part of the propellant.

### High Molecular Weight Silicone Material

The agent providing hair conditioning (e.g., dry hair combing) is a high molecular weight silicone material. References disclosing such silicones are US-A- 4,152,416; Silicon Compounds, distributed by Petrarch Systems; and Noll, Walter, Chemistry and Technology of Silicones, New York: Academic Press 1968. Also describing high molecular weight silicone materials are General Electric Silicone Rubber Product Data Sheets SE 30, SE 33, SE 54 and SE 76.

Because of the ready availability of equipment and the simplicity of the method, commercial manufacturers generally characterize high molecular weight silicones by their viscosity or in some cases by a penetration value. High molecular weight silicone materials denotes polydiorganosiloxanes having a viscosity of at least 100,000 mm$^2$•s$^{-1}$ (cSt), preferably from $10^5$ to $15 \times 10^6$. Specific examples include polydimethylsiloxane, methylphenyl-diphenyl siloxane copolymer, (polydimethylsiloxane) (methylvinylsiloxane) copolymer and poly(dimethylsiloxane) (diphenyl) (methylvinylsiloxane) copolymer and mixtures of these agents. The silicone material is present at a level of from 0.05% to 2%, preferably from 0.1% to 1% by weight of the total composition.

### Optional Components

The aerosol mousses herein can contain a variety of nonessential, optional components suitable for rendering such compositions more acceptable. Such conventional optional ingredients are well known to those skilled in the art, e.g., emulsifiers such as anionics (e.g., sodium alkyl sulfate) and nonionics (amine oxides); preservatives such as benzyl alcohol, methyl paraben, propyl paraben and imidazolidinyl urea; cationic emulsifiers/conditioners such as cetyl trimethyl ammonium chloride, stearyldimethyl benzyl ammonium chloride, and di(partially hydrogenated tallow) dimethylammonium chloride; thickeners and viscosity modifiers such as a diethanolamide of a long chain fatty acid; block polymers of ethylene oxide and propylene oxide such as Pluronic (RTM) F88 offered by BASF Wyandotte, fatty alcohols such as cetearyl alcohol, sodium chloride, sodium sulfate, and ethyl alcohol; pH adjusting agents such as citric acid, succinic acid, sodium hydroxide and triethanolamine; coloring agents such as any of the FD&C or D&C dyes; hair oxidizing (bleaching) agents such as hydrogen peroxide, perborate salts and persulfate salts; hair reducing agents such as the thioglycolates; perfume oils; chelating agents such as ethylenediamine tetracetic acid; and, among many other agents, polymer plasticizing agents such as glycerin and propylene glycol. These optional materials are generally used individually at a level of from about 0.01% to about 10%, preferably from about 0.5% to about 5% by weight of the total composition.

Another optional component preferred for use herein is a low viscosity silicone fluid to be used along with the high molecular weight silicone material in the propellant phase. Such materials may be either a polyalkyl siloxane, a polyaryl siloxane, a polyalkylaryl siloxane or a polyether siloxane copolymer. These materials preferably have a viscosity of from 5 to 10,000 $mm^2 \cdot s^{-1}$ (cSt), most preferably from 100 to 1,000 $mm^2 \cdot s^{-1}$ (cSt), at 25° C. The agent is preferably used at a level of from 0.1% to 1% by weight of the total composition.

## METHOD OF MANUFACTURE

The method of preparing the aerosol compositions of the present invention follows conventional aerosol filling procedures. The water soluble or dispersible materials (not including the silicone material) are mixed with water to form a "concentrate". This concentrate, in an appropriate amount, is placed into an aerosol container. The container is then fitted with a valve, subjected to a vacuum to rid the container of air and sealed with the valve "crimped" in place. The silicone material is then mixed with the propellant fluid and the propellant/silicone mixture is filled into the container through the valve.

## INDUSTRIAL APPLICABILITY

The present compositions are emitted from the aerosol container as a foam which is then worked through the hair with the fingers or a hair styling implement and either left on the hair or rinsed out.

The following Examples further describe and demonstrate the preferred embodiments within the scope of the present invention.

## EXAMPLE I

The following composition representative of the present invention was prepared.

| Component | Wt. % |
|---|---|
| Luviskol (RTM) K-60[2] (45% active in water) | 6.11 |
| Adogen (RTM) 470[2] (75% active in ethanol) | 0.20 |
| Schercamox (RTM) CMA[3] (38% active in water) | 0.38 |
| Preservative | 0.03 |
| Propellant A-46[4] | 10.00 |
| Silicone Gum (GE SE-76)[5] | 0.17 |
| Dimethicone Fluid (350 $mm^2 \cdot s^{-1}$ (cSt) at 25°C) | 0.33 |
| Water | qs 100.00% |

1 Offered by BASF Wyandotte having molecular weight of about 300,000

2 Di(partially hardened tallow) dimethyl ammonium chloride offered by Sherex

3 Dihydroxyethyl cocamine oxide offered by Scher Chemical Company

4 (20%/78%/2%) Propane/isobutane/n-butane offered by Phillips Petroleum Company

5 Silicone gum offered by General Electric

EXAMPLE II

The following is another composition representative of the present invention which was prepared.

| Component | Wt. % |
|---|---|
| Gafquat (RTM) 734[1] (50% active in ethanol) | 5.50 |
| Adogen (RTM) 470 (75% active in water) | 0.20 |
| Barlox (RTM) 12[2] (30% active in water) | 0.83 |
| Preservative | 0.03 |
| Propellant A-46 | 10.00 |
| Silicone Gum (GE SE-76) | 0.08 |
| Dimethicone Fluid (350 $mm^2 \cdot s^{-1}$ (cSt)) | 0.17 |
| Water | qs 100.00% |

[1] Copolymer of vinylpyrrolidone and dimethyl aminoethylmethacrylate reacted with dimethyl sulfate from GAF

[2] Dimethyl cocamine oxide offered by Lonza, Inc.

EXAMPLE III

The following is another composition of the present invention which was prepared.

| Component | Wt. % |
|---|---|
| Ultrahold 8[1] (50% active in ethanol) | 5.500 |
| Barlox (RTM) 12 (30% active in water) | 0.83 |
| SDA 40 Ethyl Alcohol | 10.000 |
| Propellant A-46 | 10.000 |

Silicone Gum (GE SE-76)        0.125
Ceteth-2        0.050
Water        qs 100.000%

[1] Neutralized form of the terpolymer of acrylic acid, ethyl acrylate and N-tertiary butyl acrylamide offered by Ciba-Geigy, Inc.

EXAMPLE IV

The following composition representative of the present invention is prepared.

| Component | Wt. % |
|---|---|
| Polyvinylpyrrolidone K-30[1] | 4.00 |
| Ditallowdimethyl Ammonium Chloride | 0.25 |
| Polyethylene Glycol 6000 Distearate | 0.80 |
| Propylene Glycol | 3.00 |
| Preservative | 0.03 |
| Propellant A-46 | 15.00 |
| Silicone Gum (GE SE-30) | 0.70 |
| Water | qs 100.00% |

[1] Offered by GAF having molecular weight of about 50,000

EXAMPLE V

The following composition of the present invention is prepared.

| Component | Wt. % |
|---|---|
| Luviskol (RTM) VA 73E[1] (50% active in ethanol) | 3.00 |
| Sodium Coconut Alkyl Sulfate | 0.50 |
| SDA 40 Ethyl Alcohol | 15.00 |
| Glycerin | 0.80 |
| Dow Corning 193 Surfactant[2] | 0.20 |
| Propellant A-46 | 7.00 |
| Silicone Gum (GE SE-76) | 0.18 |
| Water | qs 100.00% |

[1] Poly(vinylpyrrolidone/vinyl acetate) copolymer offered by BASF Wyandotte Corporation

[2] Copolyol nonionic surfactant offered by Dow Corning

In the above Examples the materials within the bracket in each Example were mixed together and filled into the aerosol container through the valve.

Any registered trade marks are acknowledged.

## Claims

1. An aerosol hair styling mousse comprising:
   a) from 0.5% to 10% of a hair setting polymer;
   b) from 0.05% to 2% of a high molecular weight silicone material;
   c) from 5% to 20% of an aerosol propellant; and
   d) water;
   characterised in that said high molecular weight silicone material has a viscosity of at least 100,000 $mm^2 \cdot s^{-1}$ (cSt) and wherein said silicone material is dissolved in said propellant prior to said propellant being put into an aerosol container.

2. An aerosol composition according to Claim 1 wherein the hair setting polymer is selected from the group oonsisting of cationic, amphoteric, nonionic and anionic polymers.

3. An aerosol composition according to Claim 1 wherein the propellant is selected from the group consisting of trichlorofluoromethane, dichlorodifluoromethane, dichlorotetrafluoroethane, monochlorodifluoromethane, trichlorotrifluoroethane, dimethyl ether, propane, isobutane, n-butane and mixtures thereof.

4. An aerosol composition according to Claim 3 wherein the hair setting polymer is selected from the group consisting of polyvinylpyrrolidone, copolymers of polyvinylpyrrolidone and other monomers and mixtures thereof.

5. A aerosol composition according to Claim 4 wherein the propellant is selected from the group consisting of propane, n-butane, isobutane and mixtures thereof.

6. An aerosol composition according to Claim 1 which in addition contains an emulsifier.

7. An aerosol composiiton according to Claim 1 which in addition contains a low molecular weight silicone fluid material having a viscosity of from 5 to 10,000 $mm^2.s^{-1}$ (cSt).

8. An aerosol composition according to Claim 5 which in addition contains an emulsifier which is cationic.

9. An aerosol composition according to Claim 8 which in addition contains a low molecular weight silicone fluid material having a viscosity of from 5 to 10,000 $mm^2.s^{-1}$ (cSt).

10. A method of styling by applying a composition according to any one of Claims 1, 4, 7 or 9 to said hair.

11. An aerosol hair styling mousse comprising:
    a) from 0.5% to 10% of a hair setting polymer;
    b) from 0.05% to 2% of a high molecular weight silicone material;
    c) from 5% to 20% of an aerosol propellant; and
    d) water;
    wherein said high molecular weight silicone material is a silicone gum and wherein said silicone material is dissolved in said propellant prior to said propellant being put into an aerosol container.

## Revendications

1. Mousse de coiffage des cheveux en aérosol, comprenant :
   a) de 0,5% à 10% d'un polymère de mise en plis;
   b) de 0,05% à 2% d'une matière de silicone de masse moléculaire élevée;
   c) de 5% à 20% d'un propulseur d'aérosol ; et
   d) de l'eau;
   caractérisée en ce que ladite matière de silicone de masse moléculaire élevée a une viscosité d'au moins 100 000 mm².s⁻¹ (cSt) et dans laquelle ladite matière de silicone est dissoute dans ledit propulseur avant que l'on mette ledit propulseur dans un récipient aérosol.

2. Composition en aérosol selon la revendication 1, dans laquelle le polymère de mise en plis est choisi dans le groupe constitué par les polymères cationiques, amphotères, non ioniques et anioniques.

3. Composition en aérosol selon la revendication 1, dans laquelle le propulseur est choisi dans le groupe constitué par le trichlorofluorométhane, le dichlorodifluorométhane, le dichlorotétrafluoroéthane, le monochlorodifluorométhane, le trichlorotrifluoroéthane, l'éther diméthylique, le propane, l'isobutane, le n-butane et leurs mélanges.

4. Composition en aérosol selon la revendication 3, dans laquelle le polymère de mise en plis est choisi dans le groupe constitué par la polyvinylpyrrolidone, les copolymères de polyvinylpyrrolidone et d'autres monomères et leurs mélanges.

5. Composition en aérosol selon la revendication 4, dans laquelle le propulseur est choisi dans le groupe constitué par le propane, le n-butane, l'isobutane et leurs mélanges.

6. Composition en aérosol selon la revendication 1, qui contient en outre un émulsifiant.

7. Composition en aérosol selon la revendication 1, qui contient en outre une matière d'huile de silicone de faible masse moléculaire ayant une viscosité de 5 à 10 000 mm².s⁻¹ (cSt).

8. Composition en aérosol selon la revendication 5, qui contient en outre un émulsifiant qui est cationique.

9. Composition en aérosol selon la revendication 8, qui contient en outre une matière d'huile de silicone de faible masse moléculaire ayant une viscosité de 5 à 10 000 mm².s⁻¹ (cSt).

10. Procédé de coiffage par application sur les cheveux d'une composition selon l'une quelconque des revendications 1, 4, 7 ou 9.

11. Mousse de coiffage des cheveux en aérosol, comprenant :
a) de 0,5% à 10% d'un polymère de mise en plis;
b) de 0,05% à 2% d'une matière de silicone de masse moléculaire élevée;
c) de 5% à 20% d'un propulseur d'aérosol ; et
d) de l'eau;
dans laquelle ladite matière de silicone de masse moléculaire élevée est une gomme de silicone et dans laquelle ladite matière de silicone est dissoute dans ledit propulseur avant que l'on mette ledit pulseur dans un récipient aérosol.

**Patentansprüche**

1. Aerosol-Frisierschaum, umfassend:
a) 0,5 % bis 10 % von eine haarfestigenden Polymer;
b) 0,05 % bis 2 % von einem Silikonmaterial mit hohem Molekulargewicht;
c) 5 % bis 20 % von einem Aerosoltreibmittel; und
d) Wasser;
dadurch gekennzeichnet, daß des genannte Silikonmaterial mit hohem Molekulargewicht eine Viskosität von mindestens 100.000 mm².s⁻¹ (cSt) besitzt und worin das genannte Silikonmaterial in dem genannten Treibmittel gelöst wird, bevor das genannte Treibmittel in einen Aerosolbehälter eingebracht wird.

2. Aerosolzusammensetzung nach Anspruch 1, worin das haarfestigende Polymer von der Gruppe bestehend aus kationischen, amphoteren, nichtionischen und anionischen Polymeren ausgewählt ist.

3. Aerosolzusammensetzung nach Anspruch 1, worin das Treibmittel von der Gruppe bestehend aus Trichlorfluormethan, Dichlordifluormethan, Dichlortetrafluorethan, Monochlordifluormethan, Trichlortrifluorethan, Dimethylether, Propan, Isobutan, n-Butan und Gemischen hievon ausgewählt ist.

4. Aerosolzusammensetzung nach Anspruch 3, worin das haarfestigende Polymer von der Gruppe bestehend aus Polyvinylpyrrolidon, Copolymeren aus Polyvinylpyrrolidon und anderen Monomeren und Gemischen hievon ausgewählt ist.

5. Aerosolzusammensetzung nach Anspruch 4, worin des Treibmittel von der Gruppe bestehend aus Propan, n-Butan, Isobutan und Gemischen hievon ausgewählt ist.

6. Aerosolzusammensetzung nach Anspruch 1, welche zusätzlich einen Emulgator enthält.

7. Aerosolzusammensetzung nach Anspruch 1, welche zusätzlich ein Silikonölmaterial mit geringem Molekulargewicht mit einer Viskosität von 5 bis 10.000 $mm^2.s^{-1}$ (cSt) enthält.

8. Aerosolzusammensetzung nach Anspruch 5, welche zusätzlich einen Emulgator enthält, welcher kationisch ist.

9. Aerosolzusammensetzung nach Anspruch 8, welche zusätzlich ein Silikonölmaterial mit geringem Molekulargewicht mit einer Viskosität von 5 bis 10.000 $mm^2.s^{-1}$ (cSt) enthält.

10. Verfahren zum Frisieren durch Aufbringen einer Zusammensetzung nach eine der Ansprüche 1, 4, 7 oder 9 auf das genannte Haar.

11. Aerosol-Frisierschaum, umfassend:
    a) 0,5 % bis 10 % von einem haarfestigenden Polymer;
    b) 0,05 % bis 2 % von eine Silikonmaterial mit hohem Molekulargewicht;
    c) 5 % bis 20 % von einem Aerosoltreibmittel; und
    d) Wasser;
    worin das genannte Silikonmaterial mit hohem Molekulargewicht ein Silikonkautschuk ist und worin das genannte Silikonmaterial in dem genannten Treibmittel gelöst wird, bevor das genannte Treibmittel in einen Aerosolbehälter eingebracht wird.